(19) Europäisches Patentamt
European Patent Office
Office européen des brevets

(11) **EP 1 185 853 B1**

(12) **EUROPEAN PATENT SPECIFICATION**

(45) Date of publication and mention
of the grant of the patent:
**15.08.2007 Bulletin 2007/33**

(21) Application number: **00937025.5**

(22) Date of filing: **01.06.2000**

(51) Int Cl.:
**G01N 21/31** (2006.01)    **A61B 5/103** (2006.01)

(86) International application number:
**PCT/GB2000/002124**

(87) International publication number:
**WO 2000/075637 (14.12.2000 Gazette 2000/50)**

(54) **METHOD OF AND APPARATUS FOR INVESTIGATING TISSUE HISTOLOGY**

VERFAHREN UND VORRICHTUNG ZUR HISTOLOGISCHEN UNTERSUCHUNG VON HAUT

PROCEDE ET APPAREIL D'ETUDE HISTOLOGIQUE DES TISSUS

(84) Designated Contracting States:
**AT BE CH CY DE DK ES FI FR GR IE IT LI LU MC NL PT SE**

(30) Priority: **04.06.1999 GB 9912908**
**28.10.1999 GB 9925414**

(43) Date of publication of application:
**13.03.2002 Bulletin 2002/11**

(73) Proprietor: **ASTRON CLINICA LIMITED**
**Toft, Cambridge CB3 7RY (GB)**

(72) Inventors:
• **Cane, Michael Roger**
**Toft,**
**Cambridge CB3 7RL (GB)**

• **Beadman, Michael Andrew**
**Nr. Royston,**
**Hertfordshire SG8 0JS (GB)**
• **Cotton, Symon D'Oyly**
**Great Gransden,**
**Sandy SG19 3AF (GB)**

(74) Representative: **Fox, Nicholas Russell Philip et al**
**BERESFORD & Co.**
**16 High Holborn**
**London WC1V 6BX (GB)**

(56) References cited:
**WO-A-96/13202        WO-A-98/22023**
**US-A- 4 241 738        US-A- 5 456 252**
**US-A- 5 701 902**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

**Description**

This invention relates to a method of and apparatus for the investigation of tissue histology. The invention has particular reference to the investigation of chromophores within layers close to the surface of such tissue, and while the invention may be applied in the investigation of laboratory tissue specimens, whether obtained from a biopsy or necropsy, it was developed with the particular intention of enabling *in vivo* observation of a subject without the need for any surgical intervention which might expose the subject, or indeed the surgeon, to the risk of infection. The invention is thus applicable especially to the investigation of epithelial tissue, such as the skin and linings of the respiratory and digestive tracts and other surfaces to which visual access may be had, such as the retina, without removing the tissue being investigated from the body of the subject.

In order to appreciate the presence of abnormalities in the tissue being examined it is first necessary to have an appreciation of the structure of normal tissue of that type.

Though the invention may be adapted for the investigation of other animal tissue, it was originally developed with particular regard to investigation of human skin conditions, and it is in that context that it will be particularly explained.

Thus, in order to appreciate the presence of abnormalities in the skin it is first necessary to have an appreciation of the structure of normal skin.

The presence and extent, including depth and concentration, of chromophores within epithelial tissue such as the skin is considered to be an important indicator of a variety of ailments and other conditions. The invention is considered to be potentially useful for the preliminary screening of patients to identify those who should be referred to an appropriate clinician for diagnosis and further to assist the clinician in diagnosis and in some embodiments to indicate whether a given treatment would be of value to the patient, and for other purposes.

The skin is divided into two main layers, the epidermis and the dermis, each of which is itself divided into several sub-layers. Starting from the deepest layer, the subcutaneous layer is overlain by a reticular layer of the dermis which is composed of coarse and dense interlacing bundles of collagen fibres ("type 1 collagen") which are intermingled with reticular fibres and elastic fibres. Over this is the papillary dermal layer which is also composed of collagen fibres but these are much finer than those of the reticular layer in that they are not bundled together. The collagen in the papillary dermis is mainly "type 3 collagen", and it constitutes connective tissue joining the epidermis and the reticular layer of the dermis. The dermis is also rich in blood vessels. The papillary dermis is located immediately beneath the epidermis and is separated from it by the basal lamina. The dermo-epidermal junction is highly irregular in profile due to dermal papillae projecting up from the dermis between rete ridges or pegs projecting down from the epidermis. It is the presence of these rete ridges or pegs and papillae which gives the skin elasticity, and their interaction also provides an anchor for the epidermis. Epithelium cells multiply continuously in a germinative layer, just above the basal lamina, to replace cells lost from the surface of the epidermis. The germinative layer, which is fed by blood vessels leading through the dermis, also contains melanocytes for the production of melanin. The epithelium cells from the germinative layer move upwards into the layer above, the spinous layer, and thence into the granular layer where the cells contain granules which are involved in the formation of keratin. It is in this granular layer that the cells of the epidermis die. Above the granular layer, is a clear and translucent layer and above that is the outermost layer, the cornified layer. This is composed of clear dead scale-like skin which is progressively lost from the surface by exfoliation.

Historically, dermatological investigations have taken place by biopsy, that is by surgical removal of samples of skin tissue followed by microscopic examination of thin sections of the skin tissue usually viewed at right angles to the skin surface. The information obtained is limited in area to the thin section, unless a number of sections is examined. Each section requires to be cut, stained and mounted onto a microscope slide, and they are therefore time consuming to prepare. Further the technique is invasive, and there may be a consequent risk of infection either at the biopsy site or from the biopsied material, or both, unless stringent precautions are taken.

In normal circumstances, the healthy epidermis is translucent and transmits light diffusely; a proportion of incident light will be absorbed in the epidermis, depending in part on the amount of melanin present in the epidermis, and a proportion will be transmitted through to the dermis. Because the papillary dermis largely consists of type 3 collagen, that is, a very fine network of collagen fibres (as low as $2\mu$m in diameter), light passing through the papillary dermis will be subject to Rayleigh scattering. A proportion of the incident light will be scattered inwards and a proportion will be back-scattered, and some of this scattered light will be remitted back through the epidermis. In the reticular dermis the fibres are of type 1 collagen, that is, they are clumped or bundled together, and they are largely parallel to the skin surface: thus they are too coarse to give rise to Rayleigh scattering, and light penetrating to the reticular dermis will continue until absorbed or deflected by some discontinuity.

Thus light remitted by the epidermis will have its spectral characteristics altered by the effects of melanin, blood and other chromophores in the skin.

The mean thickness of the papillary dermis can vary quite considerably as between one part of the body and another, for example, and in particular, the height and population density of dermal papillae tends to increase according to the stress to which a particular area of skin is habitually subjected. Thus, the thickness of the papillary dermis over a joint will tend to be greater than that over a relatively non-stressed region such as the lower back. These variations, and variations between different subjects will have a marked effect on the skin colour, but as we have previously noted (see WO 98/22023), it is possible to construct a mathematical model which allows corrections to be made for this effect. When so corrected it is notable that the colour of normal healthy human skin lies in a well defined surface area within a particular colour space, for example the CIE LMS colour space. That surface area encompasses all colours of normal healthy human skin irrespective of the amount of melanin within the skin and thus irrespective of race or degree of tanning. This approach allows parameters relating to chromophores within the skin to be measured in a more accurate and repeatable way through optical means than was permitted by previously existing techniques.

US 5701902 discloses a burn evaluation method and apparatus that allows a surgeon to make a quick evaluation of the extent and depth of a skin burn injury by employing induced ultraviolet or blue light spectroscopy and visible and infrared reflectance spectroscopy. The apparatus includes a number of light sources, a sensor and a micro-processor. The light sources emit excitation light at pre-determined wave lengths and when each is activated the sensor measures the amount of return light received. The amount of received light is then processed to obtain an evaluation of burn thickness.

According to one aspect of the present invention, there is provided apparatus for monitoring the presence of individual chromophores in a tissue sample, in accordance with claim 1.

According to a further aspect of the present invention, there is provided a method of monitoring the presence of individual chromophores in a sample of tissue in accordance with claim 28.

The apparatus and method of the present invention may be utilised for monitoring the presence of a wide variety of chromophores in the skin. It is possible to derive data relating to the presence, depth, and concentration of a wide range of chromophores, depending on measurements being made at particular wavelengths. These wavelengths may readily be selectable by light filters which may be substituted into the light path, or the analyser may be constituted by a spectroscope. The filters may be broad band filters or narrow band filters as appropriate for the analysis to be undertaken.

Examples of particular chromophores whose presence may be monitored include: melanin, blood, haemoglobin, oxy-haemoglobin, bilirubin, tattoo pigments, keratin, collagen and hair.

The control signal may be used for controlling or operating one or more of the following: a display device such as a display monitor, a printer, or a medical laser or other treatment device or apparatus.

The projected light is preferably polarised, and the remitted light is suitably cross-polarised before monitoring. This is especially suitable for monitoring the presence of chromophores beneath the epidermis. Since little scattering of light takes place in the epidermis, any cross-polarised light which is detected must have been remitted from or via the (papillary) dermis, and this allows surface effects and the effects of the epidermis to be eliminated. A similar effect can be achieved without using cross-polarised illumination by coating the surface of the skin with a transparent oil which removes direct reflections at the skin surface.

A method and apparatus according to the invention are valuable in providing information to a clinician on which the clinician may base a diagnosis or a course of treatment and the apparatus may be used for controlling the treatment and in some cases for giving an indication of whether the treatment may be effective or not.

For example, haemangioma (so-called port-wine stains, due to an abnormal distribution of blood vessels) may be diagnosed by straightforward visual inspection, and it is well known to treat the condition by laser to cauterise the blood vessels. Typically treatment for such a problem begins with the firing of a series of "test shots" by a laser at different powers to establish the minimum power necessary to cauterise the blood vessels. That power will depend on the depth and the size of those vessels, and these may vary over the extent of the lesion. The test powers are chosen by the clinician having regard to his skill and past experience. This technique suffers from a number of disadvantages. It is not very reliable since the depth and the size of the blood vessels may vary over the extent of the lesion. It is time consuming since the results of the test need to be assessed after a healing time. And the patient is left in a state of uncertainty during that time. This uncertainty is exacerbated due to the fact that a too intense laser irradiation will result in burning of the skin and consequent permanent scarring. In the cases of up to about one third of patients, the intensity of irradiation which would be required to cauterise the offending blood vessels is actually so high that there would be a serious risk of scarring and the treatment is accordingly contra-indicated.

The present invention can be used to establish not only the amount of blood present, and thus give an indication of the amount of blood vessels required to be cauterised, but also the depth of those vessels beneath the surface of the skin. The intensity of laser irradiation needed to cauterise a given amount of blood vessels is known from past experience or can be established, and the absorption characteristics of human skin in relation to the laser radiation of a given wavelength can readily be established (indeed handbooks supplied with medical lasers tend to contain this information).

Thus by making use of the present invention, light remitted from the stain can be analysed to give an indication of the melanin content of the epidermis (which governs its absorption coefficient) and of the depth and concentration of the offending blood vessels, and a prediction can be made there and then as to the intensity of laser irradiation which will be required to effect a satisfactory treatment and whether that intensity would give an acceptably low risk of permanent scarring. Further this assessment may be made at as many points over the extent of the stain as are thought necessary. Not only that, but the output signal from the apparatus may be used to control firing of a laser. Thus the power output of the laser may be varied as it is directed over the extent of the lesion. Thus the laser may be controlled to give the minimum effective power dissipation over the various increments of the lesion. Parts only of the area of the lesion could be treated if that would give a cosmetically acceptable result. And if the lesion was so severe that it was unsuitable for laser treatment, the patient could be told immediately and would not face some weeks of uncertainty.

Similar considerations apply in the case of removing tattoos by the destruction of the pigments used to make them, and the removal of moles by the destruction of melanin by which they are constituted.

The removal of hair by laser cauterisation of the hair bulb may also be controlled by apparatus according to the invention. Hair consists of keratin and its colour (and thus light energy absorption characteristics) is due to the presence of melanin. The hair bulb is located in or below the reticular dermis. Using the present invention it is possible to determine the absorption characteristics of the skin layers which would have to be penetrated by laser radiation aimed to destroy the hair bulb. The absorption characteristics of the hair bulb can be measured or calculated from a measurement of the melanin content of the hair, and the amount of energy which would have to be absorbed by the hair bulb to destroy it can also be determined, in vitro if necessary. From this information, it is possible to calculate the energy which would require to be dissipated by the laser, and it would accordingly be possible either to give a minimum energy dosage, or to predict that the minimum required dosage was so high that permanent scarring would result and that the treatment should accordingly not be carried out.

It will be appreciated that the output signal generated by the use of the invention will represent an average value over the extent of the area monitored: this will plainly be no greater than the size of the light spot which is illuminated, and its size may also be determined by the size of the photoreceptor. Means may be provided for varying the monitored area if desired, for example from a spot 0.1 mm or less (e.g. 0.01 mm) to 10 cm or more in diameter. This can be extended to provide an image of an area by providing the analysis at a number of locations. This can easily be achieved by the use of a digital camera.

To achieve these results, the system measures the light remitted from skin and compares it with the incident light at a number of wavelengths or wavelength bands. These measurements can be performed using any convenient means including filters or a spectrometer and they allow quantification of the quantities and position, including distance relative to the dermo-epidermal junction, of chromophores such as collagen, melanin, blood and keratin. Indeed these measurements can be performed on any substance assuming its absorbency and reflectivity of light are known. "Spectral measurement" is used to denote measurement of the light remitted from human skin whether by the use of a spectrometer or sub-sampling through filters which can be placed in the path of the incident or remitted light. The spectral measurements can be performed at one or more points. A collection of points whether gathered simultaneously or not can also be combined to form an image showing the measurements over the skin.

The spectral remittance of light from human skin can be calculated given knowledge of the quantity and position of substances within it. Such calculations can be performed using a variety of mathematical means including Monte Carlo modelling and the Kubelka-Munk theory, generating a value for Pn where

$$P_n(\rho_1, \rho_2, \rho_3 \ldots \rho_n, d_1, d_2, d_3 \ldots d_n, \phi_{m1}, \phi_{m2}, \phi_{m3} \ldots \phi_{mn}, d_m, \nu, \kappa) = \qquad \text{\textit{Equation 1}}$$

$$\frac{\int_0^\infty R(\rho_1, \rho_2, \rho_3 \ldots \rho_n, d_1, d_2, d_3 \ldots d_n, \phi_{m1}, \phi_{m2}, \phi_{m3} \ldots \phi_{mn}, \nu)\theta(\lambda, d_m, \kappa)^2 S(\lambda)S_{P_n}(\lambda)d\lambda}{\int_0^\infty S(\lambda)S_{P_n}(\lambda)d\lambda}$$

in which:

$P_n$ represents the calculated or measured ratio of remitted to incident light for a particular wavelength function or filter $S_{p_n}(\lambda)$ and incident light $S(\lambda)$. $\theta$ represents the light absorbed within the epidermis with dm representing the quantity of epidermal melanin and $\kappa$ the amount of keratin. R represents the ratio of light remitted from the dermis to light incident on the dermis, with $\rho_1, \rho_2, \rho_3 \ldots \rho_n$ representing the quantity of blood within n layers within the dermis, parallel with the skin surface and of thicknesses $d_1, d_2, d_3 \ldots d_n$. Within these layers, $\phi_{m1}, \phi_{m2}, \phi_{m3} \ldots \phi_{mn}$ represent the quantity of melanin within the dermis and $\nu$ the thickness of the papillary dermis. $P_n$ can also be obtained through measurements on real skin rather than by calculation.

As discussed the position within the dermis and concentration of blood is of importance to the calibration and use of medical lasers. The position of such blood will effect the remitted light from the skin generally causing the skin colour to become more purple as the depth of blood vessels increases.

To ascertain non invasive information regarding blood position and concentration the spectral composition of light remitted from skin can be ascertained as above for a representative sample of possible blood quantities and blood depths. It is also necessary to generate the possible set of remitted light measurements relating to variations in other parameters such as epidermal melanin, dermal melanin, papillary dermal thickness and keratin. As such an N dimensional search space is generated where $N$ corresponds to the number of different constituents and blood and melanin planes considered. This analysis can be extended to include any other constituents such as tattoo pigment. For analysis of skin this may have to include spectral measurements within the infrared portion of the electromagnetic spectrum as well as the visible.

Measurements of the spectral remittance from skin to be examined are then compared with the data within the $N$ dimensional search space with the closest match indicating the constituents of the skin. The data for these comparisons can either be performed as required or incorporated into pre calculated lookup tables.

Such an analysis may require a large search and it is possible for certain combinations of constituents to generate the same spectral remittance and thus multiple solutions.

Another approach is to identify those constituents of skin about which information can reliably be ascertained, quantify these and perform a transformation to the measured spectral remittance or data to which this is to be compared.

This can for instance be achieved by first adjusting for variations in the thickness of the papillary dermis in the manner described in International Patent Application published as WO 98/22023. A second quantity that must also be assessed is the quantity of melanin within the epidermis. The accuracy to which this can be assessed has a large influence on the accuracy to which the depth of blood within the dermis can be ascertained. However the presence of blood at different depths within the dermis markedly changes the remittance of light from the skin and so complicates the assessment of epidermal melanin levels by standard spectroscopic means.

A solution to this problem assumes that the quantity of epidermal melanin does not change markedly over the skin surrounding the lesion thus allowing interpolation from the surrounding areas. Such a technique may operate in certain lesions but the reliance that can be placed on the results will be lowered. A second solution is to assess the levels of epidermal melanin by a spectroscopic/light analysis method accepting any inaccuracies due to the complicating factor of blood at different depths. Following either of these techniques the N dimensional space can be reduced requiring only solutions to $P_{nr}$ to be found where

$$P_{nr}(\rho_1, \rho_2, \rho_3, d_1, d_2, d_3) = \int_0^\infty R_{nr}(\rho_1, \rho_2, \rho_3, d_1, d_2, d_3) S(\lambda) S_{P_n}(\lambda) d\lambda \quad \textit{Equation 2}$$

As discussed inaccuracies in this measurement will adversely effect the assessment of blood position within the dermis thus lowering its accuracy.

A third solution is to use a detector which is "blind" to the effect of melanin within the epidermis. Such a detector would register zero, or a constant value, when presented with melanin within the epidermis with differences in its value corresponding purely to the quantity and position of other skin constituents. Such a detector would not require transformations to data based on measures for the amount of epidermal melanin thus increasing accuracy. It is also possible to use such a detector in the generation of the $N$ dimensional search space discussed previously.

The epidermal-melanin-blind detector renders the pigment melanin effectively transparent when it lies within the epidermis of the skin. Such a detector allows viewing of structures within the skin with the obscuring effect of epidermal melanin removed. The approach outlined utilises knowledge of the variation of light absorption by melanin within the epidermis with wavelength.

At a particular wavelength λ, let the ratio of remitted to incident light from skin be $P(\lambda)$. If two wavelengths $\lambda_1$ and $\lambda_2$ are considered this leads to two values of $P$, $P(\lambda_1)$ and $P(\lambda_2)$.

Let $R_d(\lambda, \upsilon)$ represent the ratio of remitted to incident light from bloodless, melanin-free, normal dermis with a known quantity of collagen within the papillary dermis $\upsilon$. Further let $\theta(\lambda, d_m)$ represent the ratio of incident to transmitted light for melanin where dm represents the quantity of melanin. As shown in the International Patent Application published as WO 98/22023 , $P(\lambda) = \theta(\lambda, d_m)^2 R_d(\lambda, \upsilon)$ and therefore

$$P(\lambda_1) = \theta(\lambda_1, d_m)^2 R_d(\lambda_1, \upsilon) \qquad\qquad Equation\ 3$$

and

$$P(\lambda_2) = \theta(\lambda_2, d_m)^2 R_d(\lambda_2, \upsilon). \qquad\qquad Equation\ 4$$

As further shown in "The optics of human skin" The Journal of Investigative Dermatology, (R. Anderson, B. Parrish & J. Parrish), $\theta(\lambda, d_m)$ can represented in the form

$$\theta(\lambda, d_m) = e^{-d_m m(\lambda)} \qquad\qquad Equation\ 5$$

where $m(\lambda)$ is the spectral absorption coefficient of melanin. As such Equations 3 and 4 become

$$P(\lambda_1) = e^{-2d_m m(\lambda_1)} R_d(\lambda_1, \upsilon) \qquad\qquad Equation\ 6$$

and

$$P(\lambda_2) = e^{-2d_m m(\lambda_2)} R_d(\lambda_2, \upsilon) \qquad\qquad Equation\ 7$$

By taking the natural logarithm of both sides of equations 6 and 7 can be shown to equate to

$$\ln P(\lambda_1) = \ln e^{-2d_m m(\lambda_1)} + \ln R_d(\lambda_1, \upsilon) \qquad\qquad Equation\ 8$$

and

$$\ln P(\lambda_2) = \ln e^{-2d_m m(\lambda_2)} + \ln R_d(\lambda_2, \upsilon) \qquad\qquad Equation\ 9$$

which can be simplified to

$$-2d_m m(\lambda_1) = \ln P(\lambda_1) - \ln R_d(\lambda_1, \upsilon) = V_1 \qquad\qquad Equation\ 10$$

and

$$-2d_m m(\lambda_2) = \ln P(\lambda_2) - \ln R_d(\lambda_2, \upsilon) = V_2 \qquad\qquad Equation\ 11$$

The proposition for an epidermal blind detector is that $V_1 - CV_2 = 0$ where C is a constant. For this to be true:

$$-2d_m m(\lambda_1) + 2Cd_m m(\lambda_2) = 0 \qquad\qquad Equation\ 12$$

and therefore

$$C = \frac{m(\lambda_1)}{m(\lambda_2)} \qquad\qquad Equation\ 13$$

leading to

$$\ln P(\lambda_1) - \ln R_d(\lambda_1, \upsilon) - C(\ln P(\lambda_2) - \ln R_d(\lambda_2, \upsilon)) = 0 \qquad Equation\ 14$$

This discussion assumes bloodless skin where the only melanin present exists in the epidermis. For real skin however this will often not be the case, with blood, melanin in the dermis and keratin etc. being present. In this situation an extra term $E(\lambda)$ is introduced to the right hand side of Equations 8 and 9 representing the extra absorption, or indeed reflectance, introduced through the additional constituents leading to

$$\ln P(\lambda_1) = \ln e^{-2d_m m(\lambda_1)} + \ln R_d(\lambda_1, \upsilon) + \ln E(\lambda_1) \qquad Equation\ 15$$

and

$$\ln P(\lambda_2) = \ln e^{-2d_m m(\lambda_2)} + \ln R_d(\lambda_2, \upsilon) + \ln E(\lambda_2) \qquad Equation\ 16$$

and therefore

$$Equation\ 17$$
$$\ln P(\lambda_1) - \ln R_d(\lambda_1, \upsilon) - C(\ln P(\lambda_2) - \ln R_d(\lambda_2, \upsilon)) = \ln E(\lambda_1) - C \ln E(\lambda_2) = F$$

As $P(\lambda_1)$ and $P(\lambda_2)$ can be measured, $C$ is known, and $R_d(\lambda_1, \upsilon)$ and $R_d(\lambda_2, \upsilon)$ can be calculated as disclosed in the International Patent Application published as WO 98/22023, $F$ can thus be calculated. The value of F therefore indicates information about the extra terms $E(\lambda_1)$ and $E(\lambda_2)$ with

$$F = C \ln E(\lambda_2) - \ln E(\lambda_1) \qquad\qquad Equation\ 18$$

and therefore

$$e^F = \frac{E(\lambda_2)^C}{E(\lambda_1)} \qquad\qquad Equation\ 19$$

In summary, to operate the epidermal melanin blind detector measurements $P_1$ and $P_2$, where $P_1 = P(\lambda_1)$ and $P_2 = P(\lambda_2)$, of skin are made and $R_1$ and $R_2$, where $R_1 = R_d(\lambda_1, \upsilon)$ and $R_2 = R_d(\lambda_2, \upsilon)$, are calculated. $F$ is then calculated from $\ln P_1 - \ln R_1 - C(\ln P_2 - \ln R_2)$ with its value giving information about pigments and components other than epidermal melanin.

The above analysis is based on the use of two measurements at two separate frequencies. However this can be extended to broad band filters with values of $m$, the spectral absorption coefficient of melanin, calculated for each broad band filter.

As $E(\lambda)$ relates purely to the change in remitted light, whether absorbed or reflected, without reference to the quantity of epidermal melanin or papillary dermal thickness it is simple to calculate it for blood at different quantities and depths within the dermis. The measured values of $E(\lambda)$ can then be compared with these thus returning information regarding the depth of blood vessels.

This approach can be extended to analyse constituents other than blood with the removal of epidermal melanin such as the examination of keratin, tattoo pigments, dermal melanin etc. Indeed the concept of a melanin blind detector can be extended to a blood blind detector, tattoo pigment blind detector and indeed any constituent for which the light reflectance and absorbency are known.

By allowing an accurate measurement of the depth and concentration of blood vessels and other constituents, these measurements can then be used within Equation 1 thus allowing an accurate measurement of epidermal melanin.

The knowledge gained regarding the position and constituents of human skin can be utilised in Equation 1 to form a number of important measures. For instance the percentage of light at any particular wavelength, or wavelength band, which is absorbed by epidermal melanin can be ascertained. This information can then be used to calculate the likelihood of scarring occurring and thus allow the setting of a safe maximum intensity of light, whether through a laser or other illumination device, that can be applied to the skin.

Further, the intensity, or percentage of light, passing through the entire papillary dermis can be ascertained. This is calculable using an equation similar to Equation 1 to result in the ratio, T, of incident light to light passing through the entire papillary dermis being calculated for a particular wavelength function or filter $SPn(\lambda)$ and incident light $S(\lambda)$.

$$T(\rho_1, \rho_2, \rho_3 \ldots \rho_n, d_1, d_2, d_3 \ldots d_n, \phi_{m1}, \phi_{m2}, \phi_{m3} \ldots \phi_{mn}, d_m, v, \kappa) = \qquad Equation\ 20$$

$$\frac{\int_0^\infty T_d(\rho_1, \rho_2, \rho_3 \ldots \rho_n, d_1, d_2, d_3 \ldots d_n, \phi_{m1}, \phi_{m2}, \phi_{m3} \ldots \phi_{mn}, v)\theta(\lambda, d_m, \kappa)S(\lambda)S_{P_n}(\lambda)d\lambda}{\int_0^\infty S(\lambda)S_{P_n}(\lambda)d\lambda}$$

$T_d$ represents the light transmitted through the papillary dermis and can be calculated using a variety of mathematical means including Monte Carlo modelling and the Kubelka-Munk theory.

Such a measure is useful in quantifying the intensity that might impinge on a hair bulb and thus can be used to judge the efficacy of hair removal by laser or other light source.

Similarly the intensity or percentage of light that reaches blood at a particular depth can be ascertained and from this the quantity absorbed by the blood. Such a measure allows an assessment or calculation of the effectiveness of the light in treating the blood vessels.

Following the quantification of the intensity of light impinging on various structures it is possible to ascertain, or quantify, the effect such an intensity will have on these structures. This may be performed through calculation or through analysis of previous treatments or through laboratory experiments. This knowledge then allows calculation, through Equation 1, of the expected appearance of the skin at either a particular wavelength or wavelength band following the application of such light. This information could, for instance, be used to generate colour, RGB, representations of the expected result of a treatment which would be of great use in the planning of such treatment.

In preferred embodiments of the invention, the spectral analysis is undertaken at more than one, for example at least four, distinct wavelengths or wavelength bands, and in some preferred embodiments, such analysis is undertaken over the whole spectrum. In a simple construction of apparatus, a filter wheel is placed between the source of illumination, and the area of skin under inspection is successively illuminated using light of the desired different wavelengths or wavelength bands. In that case, all that is necessary is to measure the intensity of remitted light for each wavelength (band). Alternatively, white light may be used and the remitted light measured by a spectrometer to give values at each of a plurality of narrow wavelength bands covering substantially the entire spectrum.

The use of narrow wavelength bands, whether due to filtering incident or remitted light or by use of a spectrometer, has advantages in certain circumstances. For example, it may be desired to distinguish between arterial blood and venous blood. Arterial and venous blood have slightly different spectral characteristics due to the presence or absence of oxy-haemoglobin. Both oxy-haemoglobin and haemoglobin remit light strongly in the red, and their spectral curves in fact largely overlap. However, venous blood, without oxy-haemoglobin has a spectral curve with a domed peak, whereas arterial blood, due to the presence of oxy-haemoglobin has a spectral curve with twin peaks separated by a valley. The use of two narrow band filters, one at a wavelength corresponding to one or other of those peaks, and one at a wavelength corresponding to the valley in the oxy-haemoglobin spectrum and a comparison of the intensity of light remitted at those wavelengths can thus determine the presence or absence of oxy-haemoglobin and thus distinguish between venous and arterial blood.

The analysis of at least four different wavebands offers considerable advantages over previous proposals, and allows the system to be used for measuring a variety of different parameters which could not previously have been unambiguously derived from the information given. For example, it allows the offset of chromophores to be measured. By offset, we mean the distance between the dermo-epidermal boundary and the top of the population of chromophores. This is in addition to the concentration and depth of the chromophores. The problem was that the position of a spot within a three-dimensional CIE LMS colour space was not necessarily unique to a given set of measurements. The same position could be achieved by relative variation between two of the variables concerned. Previously, it had been necessary to make an estimate based on prior assumptions about the relationship between these variables. The analysis of a fourth or further wavelength band allows comparison with a notional colour space having four (or more) dimensions so that any position within that N dimensional space can be attributed to a unique depth, concentration and offset of a particular chromophore.

The present invention at least in its most preferred embodiments, enables the generation of information regarding a number of features regarding skin. To allow an accurate diagnosis of disorders of the skin, or the prognosis of treatment for such disorders, or the monitoring of healthy skin, it is important that the spatial relationship between these features can be understood. To facilitate the spatial correlation of two images, one showing the appearance of the skin and the other showing a particular feature or of two images showing different features, we have developed a technique whereby a third image is generated. Thus we also provide a method of and apparatus for showing both images together with the proportion or intensity of each adjusted through the use of a control of some means and this allows spatial correlation of the input images. For example the two original images might be supplied in overlapping relation to a monitor screen of a PC, and the two images be relatively faded in and faded out in order to change from viewing one image to another.

The display first shows an image, which may or may not be magnified, of the lesion as it actually appears to the eye or a surface microscopy view or an image taken using cross polarised illumination or an image showing a particular feature. By selecting a particular feature such as blood or areas of melanin invasion into the dermis or melanin within the epidermis etc. the display can then be faded to show this feature as an image. The fading allows a progression, or mixing, between the two views and is a convenient means of allowing a spatial correlation to be made between the features and the lesion image.

The images may be images representing the presence of particular existing features of the skin or one or more of them may be computer generated images representing the predicted effects of a treatment such as a laser irradiation treatment. For example, as mentioned above, it is possible to generate a colour representation of the expected result of a laser irradiation treatment, and it would be possible to generate one such image for each of a set of different irradiation intensities. This would enable a comparison of the different courses of treatment and would allow selection of an appropriate treatment, for example the one giving the most cosmetically acceptable result.

The analysis afforded by the present invention is also of value in the selection of the wavelength or wavelengths of any light (infra-red, visible or ultra-violet) irradiation treatment that may be indicated. For example, a knowledge of the constituents of a lesion allows a selection of a wavelength of light radiation which will be most strongly and preferentially absorbed by constituents of that lesion. Also, a knowledge of the existence and structure and composition of overlying tissue (including any discontinuities which it might contain) allows the most favourable compromise to be reached between low absorption in the overlying tissue and high absorption in the lesion to be destroyed, thus providing the most effective treatment with the lowest radiation dosage.

Thus a laser of an appropriate wavelength may be selected, and/or a variable wavelength laser may be tuned, or an appropriate filter set may be used in conjunction with a source of non-coherent radiation.

Such a technique may be applied not only to the skin as described above, but also to other epithelial or layered tissue of the human or animal body. Such tissues include the epithelium of the cervix, the lining of the mouth, epithelia of the respiratory and digestive tracts and the eye, including such specialised tissues as the sclera, cornea and retina, and epithelia of internal organs such as the liver and bladder.

The mathematical model described accounts for components such as blood, melanin and collagen, and is generally applicable to epithelial or layered tissues. It is therefore possible to predict the coloration or spectral composition of the tissue containing different amounts of these components. Characteristics of the components can thus be determined by an examination of measured spectral properties in the manner described.

For example, the cervix is covered with a stratified squamous epithelium in which the distribution of blood, collagen and melanin may be determined, and information relating to this is useful in the monitoring and diagnosis of the general state of health especially with regard to cervical cancer. A second example relates to the interior of the human eye. This includes various specialised tissues such as the sclera, cornea and retina containing blood, collagen and melanin in a layered structure. Information on the distribution of these components is useful in monitoring the health of the eye.

In each case, the technique and mathematical model described can be adapted to take account of particular or additional light absorbing or light scattering components or present in the tissue examined.

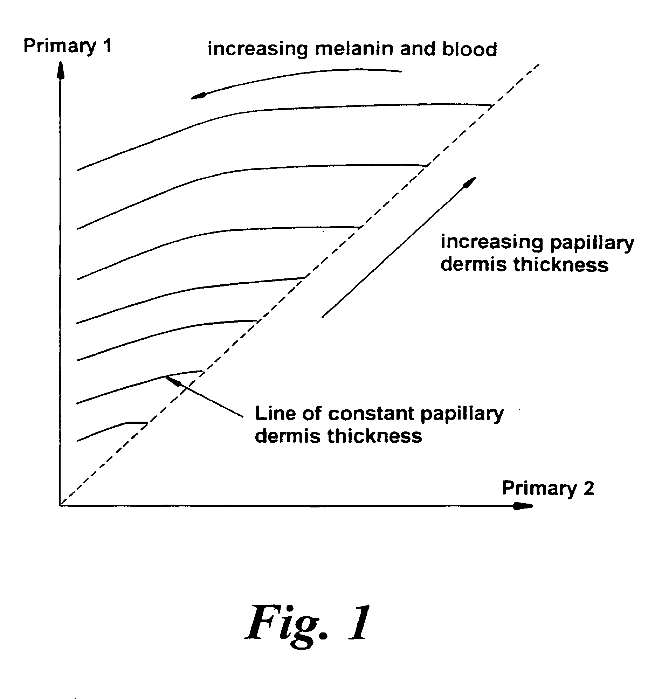

The thickness of the papillary dermis may be obtained by utilising the property of human skin to vary its remittance of red and infrared radiation with varying papillary dermis thickness. In general, there is a relationship between remittance and thickness. The fact that red or infrared radiation is also absorbed by other materials within the skin, particularly melanin and blood, is a complicating factor, but the layer thickness may still be measured by obtaining two red or infrared images, each at a different wavelength. The chosen wavelengths are not important, but one should be further into the infrared (i.e. at longer wavelength) than the other. Suitable wavelength bands are 800-1000nm and 600-800nm, in that readily available infrared films and filters may be used. The brightness of points within the image obtained at the longer wavelength is affected to a greater extent by variations in the papillary dermis thickness. Conversely, the image obtained at shorter wavelength will be affected to a greater extent by other materials such as melanin and blood. (In fact when operating sufficiently far into the infrared, say at 1100nm, the effects of melanin and blood become negligible, and it is possible to derive the necessary information using a single wavelength measurement. But this greatly increases the cost of the detection and monitoring equipment.) By predicting the brightnesses of points of differing papillary dermis thickness and amounts of epidermal melanin which absorb near-infrared radiation at the two different infrared wavelengths, a reference graph (Fig 1) can be obtained which consists of lines of constant papillary dermis thickness, wherein Primary 1 is the measurement made at the longer (800-1000nm) wavelength and Primary 2 is the measurement made at the shorter (600-800nm) wavelength. The absorption of blood within these wavelengths is very small (a hundredth of its peak value for visible wavelengths at 600-800nm and even less for 800-1000nm) and to a first approximation may be ignored. Thus, by comparing values obtained at these wavelengths with this graph, it is possible to ascertain the papillary dermis thickness. However to measure brightness at such a long infra-red wavelength e.g. 1100nm that the brightness would vary to such a negligible extent with melanin and blood content that it would effectively depend solely on the papillary dermis thickness. In such a case only one set of brightness measurements would be required.

To calculate the look-up graph shown in Fig 1 the spectral remittance of light from human skin can be calculated given knowledge of the quantity and position of substances within it. Such calculations can be performed using a variety of mathematical means including Monte Carlo modelling and the Kubelka-Munk theory generating a value for $P_n$ where

$$P_n(\rho, d_m, v) = \frac{\int_0^\infty R(\rho, v)\theta(\lambda, d_m)^2 S(\lambda) S_{P_n}(\lambda) d\lambda}{\int_0^\infty S(\lambda) S_{P_n}(\lambda) d\lambda} \qquad \text{Equation 21}$$

where $P_n$ represents the calculated or measured ratio of remitted to incident light for a particular wavelength function or filter $S_{P_n}(\lambda)$ and incident light $S(\lambda)$. $\theta$ represents the light absorbed within the epidermis with $d_m$ representing the quantity of epidermal melanin. $R$ represents the light remitted from the dermis with $\rho$ representing the quantity of blood and $v$ the thickness of the papillary dermis. $P_n$ can also be obtained through measurements on real skin rather than by calculation.

This analysis can be extended to a more general case

$$P_n(\rho_1, \rho_2, \rho_3, \ldots, \rho_n, d_1, d_2, d_3, \ldots, d_n, \phi_{m1}, \phi_{m2}, \phi_{m3}, \ldots, \phi_{mn}, d_m, v, \kappa) = \qquad \text{Equation 22}$$

$$\frac{\int_0^\infty R(\rho_1, \rho_2, \rho_3, \ldots, \rho_n, d_1, d_2, d_3, \ldots, d_n, \phi_{m1}, \phi_{m2}, \phi_{m3}, \ldots, \phi_{mn}, d_m, v)\theta(\lambda, d_m, \kappa)^2 S(\lambda) S_{P_n}(\lambda) d\lambda}{\int_0^\infty S(\lambda) S_{P_n}(\lambda) d\lambda}$$

Where $\kappa$ represent the amount of keratin and $\rho_1$, $\rho_2$, $\rho_3$, ..., $\rho_n$, the quantity of blood within n planes within the dermis parallel with the skin surface of thickness $d_1$, $d_2$, $d_3$, ..., $d_n$. Within these planes, $\phi_{m1}$, $\phi_{m2}$, $\phi_{m3}$, .... $\phi_{mn}$, represent the quantity of melanin within the dermis. As with the simple case $P_n$ can also be obtained through measurements on real skin rather than by calculation. For a detailed discussion of this technique please refer to "A non-invasive imaging system for assisting in the diagnosis of melanoma" University of Birmingham, Symon Cotton, 1998.

The above discussion relates to measurements of the thickness of the papillary dermis alone. However, according to Histology, a text and atlas, second edition, Michael Ross and Lynn Romrell, published by Williams & Wilkins, "The papillary layer consists of loose connective tissue. It is located immediately under the epidermis and is separated from it by the basal lamina. The papillary layer is a relatively thin layer extending into (and, thus, also constituting) the dermal papillae and ridges." In contrast the junction between the papillary dermis and reticular dermis is relatively smooth or at least varying with a wavelength very large in contrast to the undulations of the papillary dermis.

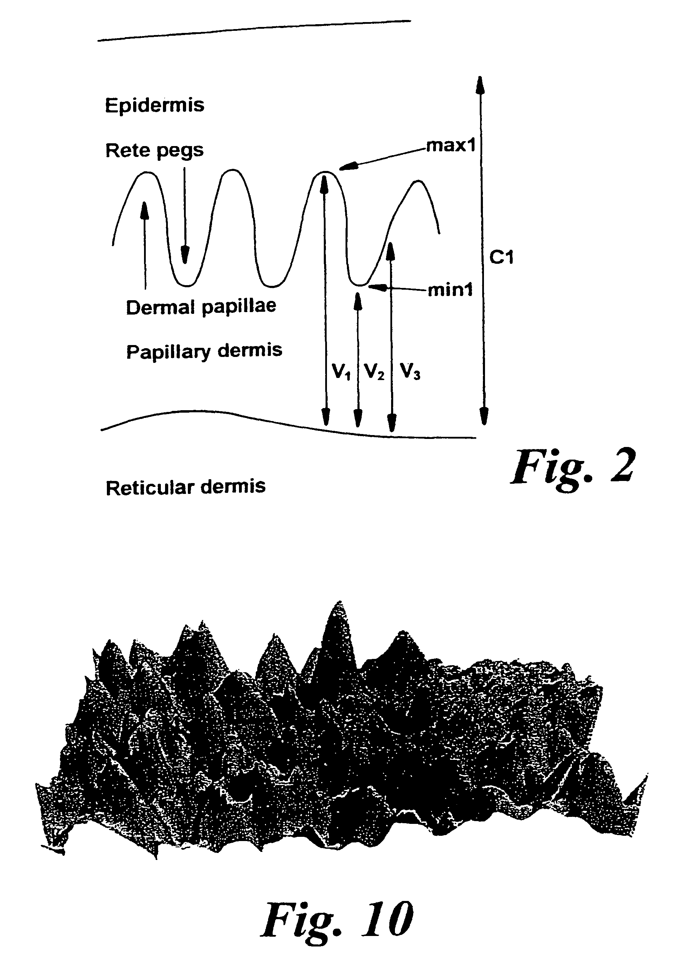

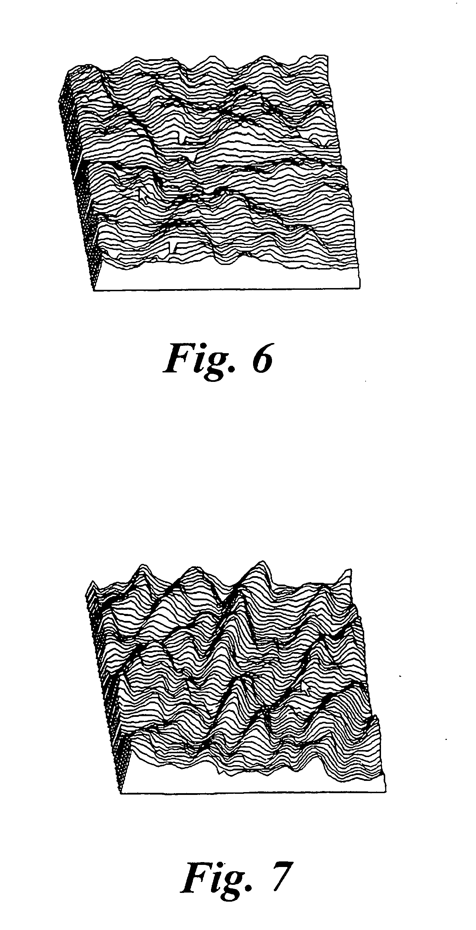

It is apparent from this as the thickness of the papillary dermis, $v$, refers to a particular sampling point, or rather the average over a sampling area, measurements taken at a variety of points return information on the thickness of the papillary dermis at these points. Further to this if it is assumed that the papillary dermis constitutes the dermal papillae and also that the junction between the papillary dermis and reticular dermis is smooth, or at least varies on a scale much larger than the dermal papillae, measurements made from a series of points $v_1$, $v_2$, $v_3$, ..., $v_n$, as shown in Fig. 2, will - if displayed spatially - show the undulations in the papillary dermis. Further measurements can be performed on the height of a particular dermal papilla by subtracting a local minimum, shown in Fig. 2 as *min1 (v2),* from a local maximum, shown in Fig. 2 as *max1* (*v1*). Examples showing dermal papillae generated using this method are shown in Figs 6 and 7.

As discussed further by *Ross* and *Romrell* "They [dermal papillae] are complemented by what appear to be a series of similar projections or evaginations, called epidermal ridges or rete ridges, which project into the dermis." It is clear from this that information regarding the rete ridges can be obtained in a similar manner as the rete ridges and dermal papillae fit together and are therefore the inverse of one another. For instance the depth of an individual peg being calculated from *max1 - minl.* To generate a three dimensional representation or two dimensional segment showing a number of rete ridges requires a calculation, *C-vn,* where *C* is a constant greater than any of the *max1-max2* measurements.

It is apparent from this that measurements of the papillary dermis thickness, $v$, measured over an area or along a line when suitably interpreted can impart information regarding the dermal papillae and rete ridges. In particular if the thickness of the papillary dermis is measured over an area or along a line and then shown graphically the undulations of the dermal papillae can be observed. As the rete ridges extend down from the epidermis filling the void between the dermal papillae it also becomes evident that the inverse of such a measure - such as a constant value minus the papillary dermis thickness - gives information regarding the rete ridges.

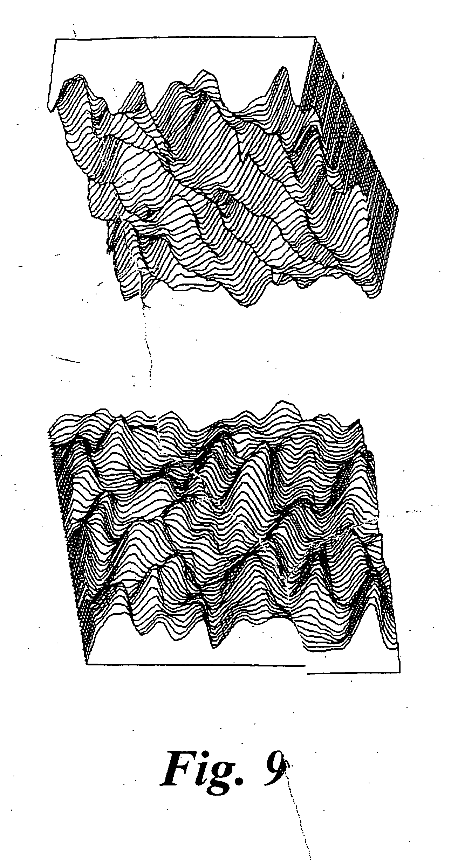

An example of this is shown in Fig. 9 where the dermal papillae pertaining to an area of skin in the shoulder region are shown rising from the dermis. In conjunction with this the rete ridges can be seen descending.

In the most preferred embodiments of the invention, means is provided for monitoring the intensity of the light remitted from a plurality of lines or a two-dimensional array of points, and preferably with a resolution of at least 20 lines or dots per mm.

This allows the production of an analogue of a three-dimensional image which can be printed or displayed on a monitor screen, and in the latter case, the use of suitable software will enable the image to be rotated so that its appearance can be viewed from a plurality of different angles.

A higher resolution may be obtained, and will indeed be necessary if inspection of a highly magnified image of the remitted light is to be obtained, but our tests have shown that a very high resolution is not necessary for many purposes. In a particularly preferred apparatus, an image of remitted light is captured using a digital camera in which use is made of a charge coupled device measuring 20 x 15 mm with a resolution of 800 $\times$ 600 pixels.

Such an image may take the form of a series of lines each of which follows the contour of the mapped surface while remaining constant in one of three orthogonal axes. Alternatively, it may comprise lines of equal contour, or it may be constituted as a continuous tone or coloured picture of the papillary surface over the area being inspected.

It is implicit in what has been stated above that no account is taken of any variations in the shape of the boundary between the papillary dermis and the reticular dermis at the intradermal junction. It is assumed that the intradermal junction is flat. In fact, as mentioned there are variations in the thickness of the papillary dermis when the presence of those papillae is discounted, but those variations are of long wavelength in comparison with variations due to the papillae and they may be neglected.

Inspection and analysis of the architecture of the dermal papillae and the epidermal rete ridges at the dermo-epidermal junction allows information to be derived which is of considerable importance to clinicians in order to assist them in diagnosing or assessing the progress of a range of dermatological phenomena.

Examples include the blistering diseases *Pemphigus vulgaris* and bullous pemphigoid. While these diseases appear clinically similar, they have very different prognoses and they require different management. *Pemphigus vulgaris* manifests itself as blisters within the thickness of the epidermis which do not distend the local dermo-epidermal boundary architecture, and it is potentially fatal with a 10% mortality rate. Bullous pemphigoid, however, gives rise to sub-epidermal blistering which does distend the local dermo-epidermal boundary architecture: prognosis is good, and the disease tends to subside over a number of months.

The dermo-epidermal boundary architecture is important in the differentiation between benign and malignant melanoma, and in identifying the presence of fibrosis within a melanoma. It is also important when assessing the extent of basal cell carcinomas and squamous cell carcinomas.

A preferred embodiment of the present invention will now be described in greater detail with reference to the accompanying diagrammatic drawings, in which:

Figure 1 is a graph showing variation of brightness with papillary dermis thickness for primaries 1 and 2 as described hereinabove,

Figure 2 shows measurements of the dermal papillae and rete ridges, also as described hereinabove,

Figures 3 to 5 are diagrammatic representations of sections through human skin such as may be revealed by conventional biopsy techniques,

Figures 6 and 7 are representations of the dermo-epidermal boundary such as may be mapped by the present invention

Figure 8 is a schematic diagram of apparatus according to this invention, and.

Figure 9 shows representations of the rete ridges (top) and dermal papillae(bottom) from an area of skin ascertained by using the present technique.

Figure 10 shows a representation of a basal cell carcinoma ascertained by using the present technique.

Figures 1 and 2 have been mentioned above.

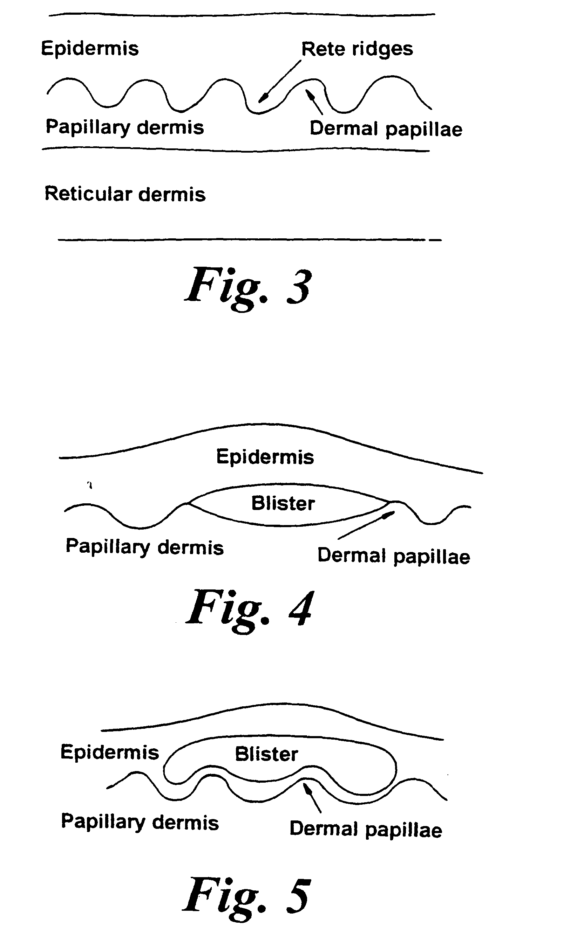

Figure 3 is an illustration of a section through normal healthy skin showing the epidermis, the papillary dermis and the reticular dermis, and shows the irregular dermo-epidermal boundary formed between the papillary dermis and the epidermis by the interpenetrating dermal papillae and the rete ridges of the epidermis.

Figure 4 is an illustration of a section through skin showing a blister due to bullous pemphigoid which gives rise to sub-epidermal blistering which distends the local dermo-epidermal boundary architecture.

Figure 5 is an illustration of a section through skin showing a blister due to *Pemphigus vulgaris* which is located within the thickness of the epidermis and which do not distend the local dermo-epidermal boundary architecture.

Figures 6 and 7 are maps of the dermo-epidermal boundary each representing a skin area of about 0.75 mm square.

In both cases the skin is normal. The shallow papillae and rete ridges shown in Figure 6 indicate that the skin is from an area which is not subject to high stress in the day-to-day life of the subject. It is in fact from the lower back. In Figure 7, the dermo-epidermal boundary is more sharply corrugated and with a shorter wavelength, indicating a greater stress to that area arising from the day-to-day life of the subject. The Figure 7 map is of skin from the shoulder. The greater degree of corrugation is associated with a greater need for elasticity and/or a greater need for a resistance to shear between the epidermis and the dermis.

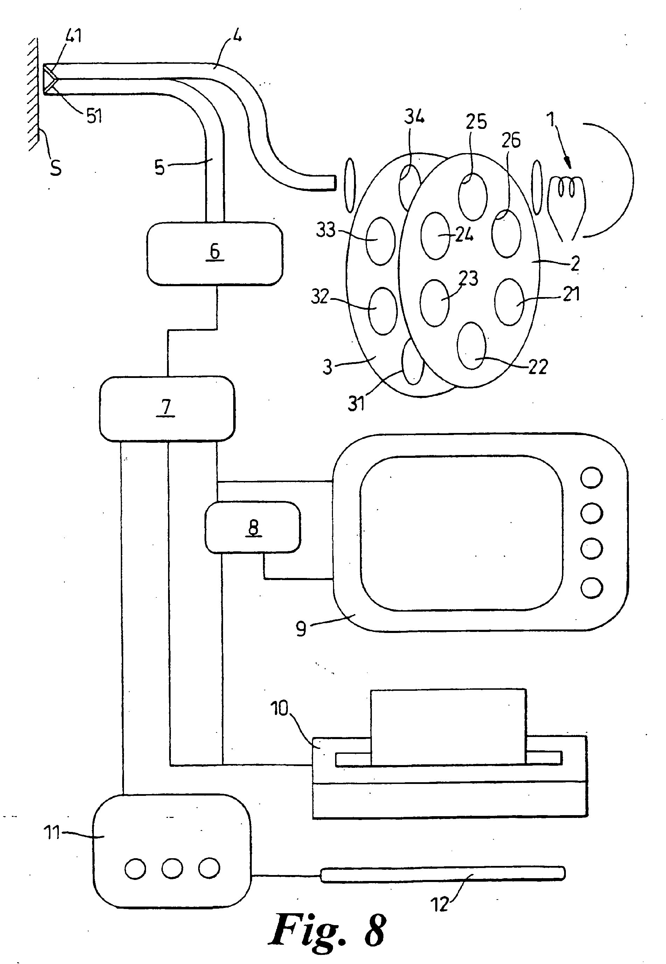

Referring now to Figure 8, a light source 1 is arranged to direct a beam of light onto a first filter wheel 2 which contains a number of holes 21 to 26 each of which may selectively be brought into the light path. One such hole is left empty for the direct transmission of light from the light source 1, while the remainder contain screens, for example of stainless steel wire gauze which serve as grey-scale filters, cutting down light-transmission without affecting its spectral characteristics. The number of grey-scale filters may be as high or as low as desired. Behind the first filter wheel 2 is a second filter wheel 3 which accommodates a number of colour filters. Four such filters 31 to 34 are shown. Again, the number of colour filters may be as high or as low as desired. One such filter may be absent for the direct transmission of light.

The colour filters would together cover as much of the spectrum as required, for example from the infra red, through to the ultra violet. For the purpose of reliably measuring the concentration of collagen within the papillary dermis, it would be possible to operate at a single wavelength of around 1050 nm, for example using a 10 nm full width-half maximum bandpass filter centred on that wavelength. This is because the absorption of light of that wavelength by melanin is negligible. However, sensors which are capable of operating in that region are expensive and it is preferred to use shorter wavelengths and to take measurements at two different wavelengths where the absorption characteristics of melanin and blood are different so that melanin and blood concentrations can be calculated and/or compensated for. It is in particular preferred to use two 10 nm full width half maximum bandpass filters respectively centred on 694 nm and 940 nm. Other colour filters may be used as desired for monitoring particular wavelengths or wavelength bands. A particularly preferred filter set includes five 10 nm full width half maximum bandpass filters respectively centred on 420, 568, 580, 694 and 940 nm, and three broad band (80 nm) filters centred on 450, 550 and 650 nm.

The reason for using grey-scale filters is that a rather high intensity light source is required for obtaining measurements in the infra-red region due to the low transmission of colour filters passing light of such wavelengths. In fact we presently prefer to use a xenon light source rated at 300 Watt. Direct transmission of such light, or transmission through for example a yellow filter could burn out a sensor suitable for monitoring in the infra-red. The use of a suitably selected set of grey-scale filters enables a single light source and a single sensor to be used, and this simplifies the apparatus and keeps costs down. A suitable set of grey-scale filters comprises those passing 50%, 10% and 1% of incident light

The light is passed to a bundle of optical fibres 4 through which it is transmitted to the skin S of the patient, or even to an appropriate photographic image of that skin, via a polarising filter 41. Remitted light is carried back through a second polarising filter 51 and a second bundle of optical fibres 5 to a photo-receptor unit 6. In other embodiments, the optical fibres 4, 5 run along an endoscope appropriate for the *in vivo* examination of internal epithelial tissue.

The two polarising filters 41, 51 are set so that their respective planes of polarisation are at right angles, to eliminate specularly reflected light.

The photo-receptor unit 6, which may simply measure the intensity of the remitted light where a series of colour filters is used as illustrated, emits a signal to a comparator 7 which may be constituted as a suitably programmed PC.

As previously mentioned, the photo-receptor is suitably a CCD array, for example a $20 \times 15$ mm array adapted to resolve 800 x 600 pixels.

The use of the bundles of optical fibres adds greatly to the convenience of use of the apparatus since a relatively small unit at the end of a flexible lead may thereby be brought to the patient's skin S: thus the physical posture of the subject during measurement is largely irrelevant and he or she may be made as comfortable as possible.

The comparator 7 is arranged to process the signals received which relate to the intensity of light remitted at the wavelengths 694 nm and 940 nm, and to derive therefrom a signal proportional to the concentration of collagen within the papillary dermis.

The comparator 7 is suitably arranged to supply the results for each pixel monitored via a processor 8 to a display monitor 9 and/or to a printer 10. The processor 8 is arranged to take the signal proportional to the collagen concentration and to use that signal as a measure of altitude to generate a relief map for printing or display. The processor 8 is suitably programmed to allow rotation of the display of the relief map. Examples of such relief maps which show the architecture of the dermo-epidermal boundary constitute Figures 4 and 5 of this specification.

The present invention at least in its most preferred embodiments, enables the generation of information regarding a number of features of any skin being examined. To allow an accurate diagnosis of disorders of the skin, or the prognosis of treatment for such disorders, or the monitoring of healthy skin, it is important that the spatial relationship between these features can be understood. Such an understanding of the dermo-epidermal boundary is greatly facilitated by preferred embodiments of the present invention in which such a map is provided. Such a map may be provided within seconds. Previously, examination by biopsy could reveal contours along a single line section, or more than one section if sufficient biopsy material was taken, but it would be at least several hours and could well be several days before the results were available to the clinician.

The comparator 7 may also receive signals relating to the intensity of light remitted in the red, yellow and blue regions of the spectrum, and of remitted white light. The comparator is arranged to assign a notional position in a colour space according to co-ordinates represented by these red, yellow and blue values and to note that position having regard to the infra-red value. Instead of measurements over the three primary wavebands, other filters may be provided so that the visible spectrum is split up into four or more wavebands. This establishes four or more co-ordinates, and the comparator may thus assign a notional position in a colour space having four or more dimensions. That position can be unique as representing the presence, depth, offset and concentration of any one or more of a range of chromophores within the skin. The comparator is suitably arranged to supply these results to a display monitor 9 and/or to a printer 10, and it may be arranged to pass control signals to the power supply 11 of a medical laser 12 or other source of radiation whether coherent or non-coherent.

The monitor 9 may be and preferably is provided with a touch screen whereby any of the various operational or programming steps may be initiated.

In some preferred embodiments of the invention, a mask is provided to surround the area of skin being illuminated and remit light back to the photoreceptor 6. The incorporation of a standard reflector into such a mask simplifies calibration of the apparatus.

Thus by making use of the invention it is possible to obtain images which correspond to: (a) the visual appearance of the skin surface; (b) the architecture of the dermo-epidermal boundary; and (c) the presence of any chromophore within the skin, including its depth and concentration, and an indication of its nature.

To facilitate the spatial correlation of two or more of such images, for example one showing the appearance of the skin and another showing a particular feature, or of two images showing different features, we have developed a technique whereby a further image is generated. Thus we also provide a method of and apparatus for showing both images together with the proportion or intensity of each adjusted through the use of a control of some means and this allows spatial correlation of the input images. For example the two original images might be supplied in overlapping relation to a monitor screen of a PC, and the two images be relatively faded in and faded out in order to change from viewing one image to another. This allows correlation between the surface appearance of skin and any underlying feature which might have given rise to that appearance. It is of particular interest in the examination of any lesion in the skin.

The display first shows an image, which may or may not be magnified, of the lesion as it actually appears to the eye or a surface microscopy view or an image taken using cross polarised illumination or an image showing a particular feature. By selecting a particular feature such as blood or areas of melanin invasion into the dermis or melanin within the epidermis etc. the display can then be faded to show this feature as an image. The fading allows a progression, or mixing, between the two views and is a convenient means of allowing a spatial correlation to be made between the features and the lesion image.

The images may be images representing the presence of particular existing features of the skin or one or more of them may be computer generated images representing the predicted effects of a treatment such as a laser irradiation treatment. For example, as mentioned above, it is possible to generate a colour representation of the expected result of a laser irradiation treatment, and it would be possible to generate one such image for each of a set of different irradiation intensities. This would enable a comparison of the different courses of treatment and would allow selection of an appropriate treatment, for example the one giving the most cosmetically acceptable result.

The analysis afforded by the present invention is also of value in the selection of the wavelength or wavelengths of any light (infra-red, visible or ultra-violet) irradiation treatment that may be indicated. For example, a knowledge of the constituents of a lesion allows a selection of a wavelength of light radiation which will be most strongly and preferentially absorbed by constituents of that lesion. Also, a knowledge of the existence and structure and composition of overlying tissue (including any discontinuities which it might contain) allows the most favourable compromise to be reached between low absorption in the overlying tissue and high absorption in the lesion to be destroyed, thus providing the most effective treatment with the lowest radiation dosage.

Thus a laser of an appropriate wavelength may be selected, and/or a variable wavelength laser may be tuned, or an appropriate filter set may be used in conjunction with a source of non-coherent radiation.

As illustrated by Fig. 10, the dermo-epidermal boundary architecture is important *inter alia* for assessing the extent of basal cell carcinomas.

Figure 10 is a map of the dermo-epidermal boundary which includes a part affected by such a carcinoma. The contrast between well developed and distinct papillae of healthy skin to the left of the Figure and the area of almost destroyed papillae at the upper right section of the Figure is well marked and clearly shows the boundary of such a carcinoma. The information imparted by such a map of the dermo-epidermal boundary is plainly of value in assisting diagnosis and in the planning of surgical excision boundaries.

**Claims**

1. Apparatus for monitoring the presence of individual chromophores in a tissue sample, said apparatus comprising:

   a light source (1) operable to illuminate an area of a tissue sample with different wavelengths of light;
   a photo-receptor (6) arranged to receive light re-emitted by an area of a tissue sample illuminated by said light source (1); and
   a spectroscopic analyser (7,8) operable to utilise the spectral characteristics of re-emitted light received by said photo-receptor (6) and the spectral characteristics of light projected by said light source (1) to identify the presence of said one or more chromophores within the area of the illuminated issue sample from which re-emitted light is received by said photo-receptor (6), **characterized in that** said spectroscopic analyser (7,8) comprises:

   records of the spectral characteristics of light re-emitted by reference samples of tissue containing identified chromophores;
   said spectroscopic analyser (7,8) being arranged to compare the spectral characteristics of re-emitted light received by said photo-receptor (6) with said records to identify the record including spectral characteristics most closely matching the characteristics of said re-emitted light received by said photo-receptor (6) and to output as an identification of individual chromophores present in the area of the illuminated issue sample from which re-emitted light is received by said photo-receptor (6) an identification of the chromophores associated with said spectral characteristics by said record.

2. Apparatus in accordance with claim 1, wherein said records comprise records of the spectral characteristics of light remitted by reference samples of epithelial or epithelial and sub-epithelial tissue.

3. Apparatus in accordance with claim 2, wherein said records comprise records of the spectral characteristics of light remitted by reference samples of skin.

4. Apparatus in accordance with any preceding claim, wherein said records comprise records of the spectral characteristics of light remitted by reference samples of normal healthy tissue.

5. Apparatus in accordance with any preceding claim, wherein said records comprise records of calculated estimated spectral characteristics of light re-emitted by samples of tissue containing identified chromophores.

6. Apparatus in accordance with any preceding claim wherein said light source (1) is operable to selectively illuminate an area of a tissue sample with different wavelengths of light.

7. Apparatus in accordance with claim 6, wherein said light source (1) further comprises a set of filters (31-34) for selective substitution into the tissue-incident light path in order to selectively illuminate an area of tissue with different wavelengths of light.

8. Apparatus in accordance with any preceding claim, wherein said light source (1) further comprises means (41) for polarising the light which illuminates said area of tissue.

9. Apparatus in accordance with claim 8, further comprising means (51) for cross-polarising light remitted from said area of tissue before it is received by said photo-receptor (6).

10. Apparatus in accordance with any preceding claim, wherein said light source (1) is operable to illuminate said area of tissue with light having a wavelength in excess of 600 nm.

11. Apparatus in accordance with any preceding claim, wherein said photo-receptor (6) is operable to detect re-emitted light having wavelengths in the 800 to 1000 nm band and the 600 to 800 nm band.

12. Apparatus in accordance with any preceding claim, wherein said photo-receptor (6) is operable to receive light remitted from an array of points and said spectroscopic analyser (7, 8) is operable to identify the presence of one or more individual chromophores at each of said points in said array.

13. Apparatus in accordance with claim 12, wherein said photo-receptor (6) is operable to receive light remitted from points with a resolution of at least 20 lines or dots per mm.

14. Apparatus in accordance with any preceding claim wherein said spectroscopic analyser (7,8) is operable to determine on the basis of the spectral characteristics of said received re-emitted light the depth of papillary dermis of a tissue sample being monitored and to identify the presence of said one or more individual chromophores within the area of the illuminated issue sample from which re-emitted light is received by said photo-receptor (6) utilising the spectral characteristics of said received re-emitted light and said determined depth of papillary dermis.

15. Apparatus in accordance with any preceding claim wherein said light source (1), photo-receptor (6) and spectroscopic analyser (7,8) are together adapted to generate a signal indicative of the presence of chromophores which is substantially independent to the variation of spectral characteristics of re-emitted light arising due to the presence of a predetermined chromophore.

16. Apparatus in accordance with claim 15 wherein said light source (1) and photo-receptor (6) are arranged to project and detect light at wavelengths which are substantially unaffected by the presence of a predetermined chromophore.

17. Apparatus in accordance with claim 15 or 16 wherein said spectroscopic analyser (7,8) is arranged to process data indicative of the spectral characteristics of re-emitted light received by said photo-receptor (6) to generate signals which are indicative of the presence of chromophores which are substantially independent of the variation in spectral characteristics arising due to the presence of a predetermined chromophore.

18. Apparatus in accordance with claim 15, 16 or 17 wherein said predetermined chromophore comprises any of melanin, blood and tattoo pigments.

19. Apparatus according to any preceding claim, further comprising a light guide of which a least part is flexible provided for conducting light between said light source (1), said tissue sample and said photo-receptor (6).

20. Apparatus according to claim 16, wherein said light guide comprises an endoscope.

21. Apparatus according to claim 17, wherein said light guide terminates in a head adapted for placing against an area of skin.

22. Apparatus according to any preceding claim, wherein means is provided for varying the size of the area of tissue from which re-emitted light is received.

23. Apparatus in accordance with any preceding claim, wherein said light source (1) is operable to project light which either has a wavelength sufficiently far into the infra-red that its absorption by melanin and blood is negligible, or which has at least two wavelengths of which at least one is in excess of 600 nm, wherein said spectroscopic analyser (7,8) is operable to process data indicative of the spectral characteristics of re-emitted light received by said photo receiver to determine variations in spectral characteristics arising independently of variations due to the presence of melanin and blood and to utilise said determined independent variations in spectral characteristics to generate a signal indicative of the concentration of collagen within the papillary dermis of a tissue sample being monitored.

24. Apparatus in accordance with any preceding claim further comprising means for generating control signals on the basis of the determination of the presence of one or more chromophores in a tissue sample by said spectroscopic analyser (7,8), wherein said control signals are operable to control any of a display monitor (9), a printer (10), or a medical laser (12) or other treatment device or apparatus.

25. Use of apparatus according to any preceding claim for deriving data relating to the presence, depth, and concentration of chromophores and creating and displaying a map thereof.

26. Use of apparatus according to any of claims 1 to 24 for deriving data relating to the presence, depth, and concentration of any chromophore selected from the group consisting of: melanin, blood, haemoglobin, oxy-haemoglobin, bilirubin, tattoo pigments or dyestuffs, keratin, collagen and hair.

27. Use of apparatus according to any of claims 1 to 24 for mapping the extent of a basal cell carcinoma.

28. A method of monitoring the presence of individual chromophores in a sample of tissue, comprising:

storing records of the spectral characteristics of light re-emitted by reference samples of tissue containing identified chromophores;
illuminating an area of a tissue sample with different wavelengths of light using a light source (1) ;
receiving light re-emitted by the illuminated area of tissue at a photo-receptor (6);
comparing the spectral characteristics of re-emitted light received by said photo-receptor (6) with said records to identify the record including spectral characteristics most closely matching the characteristics of said re-emitted light received by said photo-receptor (6); and
outputting as an identification of the individual chromophores present in the area of the illuminated tissue sample from which re-emitted light is received by said photo-receptor (6) an identification of the chromophores associated with said spectral characteristics by said record.

29. A method according to claim 28, wherein said records comprise records of the spectral characteristics of light remitted by reference samples of epithelial or epithelial and sub-epithelial tissue.

30. A method according to claim 29, wherein said records comprise records of the spectral characteristics of light remitted by reference samples of skin.

31. A method according to claim 28, 29 or 30, wherein said records comprise records of the spectral characteristics of light remitted by reference samples of normal healthy tissue.

32. A method according to any of claims 28-31, wherein said records comprise records of calculated estimated spectral characteristics of light re-emitted by samples of tissue containing identified chromophores.

33. A method according to any of claims 28-32, further comprising the steps of:

deriving data relating to the presence, depth, and concentration of chromophores; and
creating and displaying a map thereof.

34. A method according to any of claims 28-33, comprising: deriving data relating to the presence, depth, and concentration of any chromophore selected from the group consisting of: melanin, blood, haemoglobin, oxy-haemoglobin, bilirubin, tattoo pigments or dyestuffs, keratin, collagen and hair.

35. A method according to any of claims 28-34 which further comprising: passing projected light and/or the remitted light through one or more filters and spectroscopically analysing the remitted light such that the result of the analysis is independent of the presence of a predetermined chromophore in the tissue.

36. A method according to claim 35 wherein said light source (1) and photo-receptor (6) are arranged to project and detect light at wavelengths which are substantially unaffected by the presence of said predetermined chromophore.

37. A method according to claim 35 or 36 further comprising:

processing data indicative of the spectral characteristics of re-emitted light received by said photo-receptor (6) to generate signals which are indicative of the presence of chromophores which are substantially independent of the variation in spectral characteristics arising due to the presence of said predetermined chromophores; and comparing said processed data with said records to identify the record having the most closely corresponding spectral data.

38. A method according to claim 35, 36 or 37 wherein said predetermined chromophore comprises any of melanin, blood and tattoo pigments.

39. A method according to any of claims 28-38, further comprising:

determining the depth of papillary dermis of a tissue sample being monitored on the basis of the spectral characteristics of said received re-emitted light; and
identifying the presence of individual chromophores within the area of the illuminated issue sample from which re-emitted light is received utilising the spectral characteristics of said received re-emitted light and said determined depth of papillary dermis

**Patentansprüche**

1. Vorrichtung zum Überwachen des Vorliegens einzelner Chromophore in einer Gewebeprobe, die Vorrichtung umfassend:

eine Lichtquelle (1), die dazu dient, eine Fläche einer Gewebeprobe mit Licht verschiedener Wellenlängen zu beleuchten,
einen Photoempfänger (6), der dazu ausgelegt ist, Licht zu empfangen, das von einer Fläche einer durch die Lichtquelle (1) beleuchteten Gewebeprobe zurückgesendet worden ist, und
eine spektroskopische Analyseeinrichtung (7, 8), die dazu dient, die Spektraleigenschaften des vom Photoempfänger (6) empfangenen zurückgesendeten Lichts und die Spektraleigenschaften des von der Lichtquelle (1) projezierten Lichts dazu zu verwenden, das Vorliegen eines oder mehrerer Chromophore innerhalb der Fläche der beleuchteten Gewebeprobe, von der zurückgesendetes Licht durch den Photoempfänger (6) empfangen wird, zu identifizieren, **dadurch gekennzeichnet, daß** die spektroskopische Analyseeinrichtung (7, 8) umfaßt:

Einträge der Spektraleigenschaften von Licht, das von identifizierten Chromophoren enthaltenden Referenzgewebeproben zurückgesendet wurde,

wobei die spektroskopische Analyseeinrichtung (7, 8) dazu ausgelegt ist, die Spektraleigenschaften des vom Photoempfänger (6) empfangenen, zurückgesendeten Lichts mit den Einträgen zu vergleichen, um den Eintrag zu identifizieren, der Spektraleigenschaften enthält, die am meisten mit den Eigenschaften des vom Photoempfänger (6) empfangenen zurückgesendeten Lichts übereinstimmen, und eine Identifikation der zu den Spektraleigenschaften des Eintrags gehörigen Chromophore als eine Identifikation einzelner Chromophore auszugeben, die in der Fläche der beleuchteten Gewebeprobe vorliegen, von der zurückgesendetes Licht vom Photoempfänger (6) empfangen worden ist.

2. Vorrichtung nach Anspruch 1, wobei die Einträge Einträge der Spektraleigenschaften von Licht umfassen, das von Referenzproben von Epithelgewebe oder von Epithel- und Subepithelgewebe zurückgesendet worden ist.

3. Vorrichtung nach Anspruch 2, wobei die Einträge Einträge der Spektraleigenschaften von Licht umfassen, das von Referenzproben von Haut zurückgesendet worden ist.

4. Vorrichtung nach einem der vorhergehenden Ansprüche, wobei die Einträge Einträge der Spektraleigenschaften von Licht umfassen, das von Referenzproben normalen gesunden Gewebes zurückgesendet worden ist.

5. Vorrichtung nach einem der vorhergehenden Ansprüche, wobei die Einträge Einträge von berechneten geschätzten Spektraleigenschaften von Licht umfassen, das von Proben von Gewebe mit identifizierten Chromophoren zurückgesendet worden ist.

6. Vorrichtung nach einem der vorhergehenden Ansprüche, wobei die Lichtquelle (1) dazu dient, wahlweise eine Fläche einer Gewebeprobe mit Licht verschiedener Wellenlängen zu beleuchten.

7. Vorrichtung nach Anspruch 6, wobei die Lichtquelle (1) ferner einen Satz von Filtern (31 bis 34) zum wahlweisen Einsatz in den auf das Gewebe einfallenden Lichtweg umfaßt, um wahlweise eine Gewebefläche mit Licht verschiedener Wellenlängen zu beleuchten.

8. Vorrichtung nach einem der vorhergehenden Ansprüche, wobei die Lichtquelle (1) ferner eine Einrichtung (41) zum Polarisieren des Lichts umfaßt, das die Gewebefläche beleuchtet.

9. Vorrichtung nach Anspruch 8, ferner umfassend eine Einrichtung (51) zum Kreuzpolarisieren von Licht, das von der Gewebefläche zurückgesendet wird, bevor es vom Photoempfänger (6) empfangen wird.

10. Vorrichtung nach einem der vorhergehenden Ansprüche, wobei die Lichtquelle (1) dazu dient, die Gewebefläche mit Licht zu beleuchten, das eine Wellenlänge oberhalb von 600 nm hat.

11. Vorrichtung nach einem der vorhergehenden Ansprüche, wobei der Photoempfänger (6) dazu dient, zurückgesendetes Licht mit Wellenlängen im Bereich von 800 bis 1000 nm und im Bereich von 600 bis 800 nm zu detektieren.

12. Vorrichtung nach einem der vorhergehenden Ansprüche, wobei der Photoempfänger (6) dazu dient, Licht zu empfangen, das von einer Anordnung von Punkten zurückgesendet worden ist, und die spektroskopische Analyseeinrichtung (7, 8) dazu dient, das Vorliegen eines oder mehrerer einzelner Chromophore an jedem der Punkte in der Anordnung zu identifizieren.

13. Vorrichtung nach Anspruch 12, wobei der Photoempfänger (6) dazu dient, Licht zu empfangen, das von Punkten mit einer Auflösung von zumindest 20 Zeilen oder Dots pro mm zurückgesendet worden ist.

14. Vorrichtung nach einem der vorhergehenden Ansprüche, wobei die spektroskopische Analyseeinrichtung (7, 8) dazu dient, auf der Grundlage der Spektraleigenschaften des empfangenen zurückgesendeten Lichts die Tiefe papillarer Dermis einer überwachten Gewebeprobe zu bestimmen und das Vorliegen eines oder mehrerer einzelner Chromophore innerhalb der Fläche der beleuchteten Gewebeprobe, von der zurückgesendetes Licht vom Photoempfänger (6) empfangen wird, mittels der Spektraleigenschaften des empfangenen zurückgesendeten Lichts und der bestimmten Tiefe der papillaren Dermis zu identifizieren.

15. Vorrichtung nach einem der vorhergehenden Ansprüche, wobei die Lichtquelle (1), der Photoempfänger (6) und die spektroskopische Analyseeinrichtung (7, 8) zusammen dazu ausgelegt sind, ein Signal zu erzeugen, das das Vorliegen von Chromophoren anzeigt und das im wesentlichen unabhängig von der Variation spektraler Eigenschaften von zurückgesendetem Licht ist, die aufgrund des Vorliegens eines vorbestimmten Chromophors auftreten.

16. Vorrichtung nach Anspruch 15, wobei die Lichtquelle (1) und der Photoempfänger (6) dazu ausgelegt sind, Licht mit Wellenlängen, die im wesentlichen vom Vorliegen eines vorbestimmten Chromophors unbeeinflußt sind, zu projezieren und zu detektieren.

**17.** Vorrichtung nach Anspruch 15 oder 16, wobei die spektroskopische Analyseeinrichtung (7, 8) dazu ausgelegt ist, Daten zu verarbeiten, die die Spektraleigenschaften von vom Photoempfänger (6) empfangenen, zurückgesendeten Licht anzeigen, um Signale zu erzeugen, die das Vorliegen von Chromophoren anzeigen, die im wesentlichen unabhängig von der Variation der Spektraleigenschaften, die aufgrund des Vorliegens eines vorbestimmten Chromophors auftreten, sind.

**18.** Vorrichtung nach Anspruch 15, 16 oder 17, wobei der vorbestimmte Chromophor Melanin, Blut oder Tätowierungspigmente umfaßt.

**19.** Vorrichtung nach einem der vorhergehenden Ansprüche, ferner umfassend einen Lichtleiter, der zumindest teilweise flexibel ist und zum Führen von Licht zwischen der Lichtquelle (1), der Gewebeprobe und dem Photoempfänger (6) vorgesehen ist.

**20.** Vorrichtung nach Anspruch 16, wobei der Lichtleiter ein Endoskop umfaßt.

**21.** Vorrichtung nach Anspruch 17, wobei der Lichtleiter in einem Kopfende endet, das dazu ausgelegt ist, gegen eine Hautfläche plaziert zu werden.

**22.** Vorrichtung nach einem der vorhergehenden Ansprüche, wobei eine Einrichtung zum Ändern der Größe der Gewebefläche, von der zurückgesendetes Licht empfangen wird, vorgesehen ist.

**23.** Vorrichtung nach einem der vorhergehenden Ansprüche, wobei die Lichtquelle (1) dazu dient, Licht zu projezieren, das entweder eine Wellenlänge hat, die hinreichend weit in den Infrarotbereich reicht, so daß seine Absorption durch Melanin und durch Blut vernachlässigbar ist, oder das mindestens zwei Wellenlängen aufweist, von denen zumindest eine oberhalb von 600 nm liegt, wobei die spektroskopische Analyseeinrichtung (7, 8) dazu dient, Daten zu verarbeiten, die die Spektraleigenschaften von vom Photoempfänger empfangenen, zurückgesendeten Licht anzeigen, um Variationen von Spektraleigenschaften zu bestimmen, die unabhängig von Variationen aufgrund des Vorliegens von Melanin und Blut auftreten, und die bestimmten unabhängigen Variationen von Spektraleigenschaften zu verwenden, um ein Signal zu erzeugen, das die Konzentration von Collagen innerhalb der papillaren Dermis einer überwachten Gewebeprobe anzeigt.

**24.** Vorrichtung nach einem der vorhergehenden Ansprüche, ferner umfassend eine Einrichtung zum Erzeugen von Steuerungssignalen auf der Grundlage der Bestimmung des Vorliegens eines oder mehrerer Chromophore in einer Gewebeprobe durch die spektroskopische Analyseeinrichtung (7, 8), wobei die Steuerungssignale dazu dienen, einen Anzeigemonitor (9), einen Drucker (10) oder einen medizinischen Laser (12) oder andere Behandlungsgeräte oder -vorrichtungen zu steuern.

**25.** Verwendung einer Vorrichtung nach einem der vorhergehenden Ansprüche zum Herleiten von Daten, die sich auf das Vorliegen, die Tiefe und die Konzentration von Chromophoren beziehen, und zum Erschaffen und Anzeigen einer Karte davon.

**26.** Verwendung einer Vorrichtung nach einem der Ansprüche 1 bis 24 zum Herleiten von Daten, die sich auf das Vorliegen, die Tiefe und die Konzentration von Chromophoren beziehen, die aus der folgenden Gruppe ausgewählt sind: Melanin, Blut, Haemoglobin, Oxyhaemoglobin, Bilirubin, Tätowierungspigmenten oder -farbstoffen, Keratin, Collagen und Haar.

**27.** Verwendung einer Vorrichtung nach einem der Ansprüche 1 bis 24 zum Abbilden des Ausmaßes eines Basalzellenkarzinoms.

**28.** Verfahren zum Überwachen des Vorliegens einzelner Chromophore in einer Gewebeprobe, umfassend:

Speichern von Einträgen der Spektraleigenschaften von Licht, das von Referenzproben von Gewebe mit identifizierten Chromophoren zurückgesendet worden ist,
Beleuchten einer Fläche einer Gewebeprobe mit Licht verschiedener Wellenlängen mittels einer Lichtquelle (1),
Empfangen von Licht, das von der beleuchteten Gewebefläche zurückgesendet worden ist, an einem Photoempfänger (6),

Vergleichen der Spektraleigenschaften des vom Photoempfänger (6) empfangenen, zurückgesendeten Lichts mit den Einträgen, um den Eintrag zu identifizieren, der spektrale Eigenschaften enthält, die am meisten mit den Eigenschaften des vom Photoempfänger (6) empfangenen zurückgesendeten Licht übereinstimmen, und Ausgeben einer Identifikation der zu den Spektraleigenschaften des Eintrags gehörenden Chromophore als eine Identifikation der einzelnen Chromophore, die in der Fläche der beleuchteten Gewebeprobe vorhanden sind, von der zurückgesendetes Licht vom Photoempfänger (6) empfangen worden ist.

29. Verfahren nach Anspruch 28, wobei die Einträge Einträge der Spektraleigenschaften von Licht umfassen, das von Referenzproben von Epithelgewebe oder von Epithel- und Subepithelgewebe ausgesendet worden ist.

30. Verfahren nach Anspruch 29, wobei die Einträge Einträge der Spektraleigenschaften von Licht umfassen, das von Referenzproben von Haut zurückgesendet worden ist.

31. Verfahren nach Anspruch 28, 29 oder 30, wobei die Einträge Einträge der Spektraleigenschaften von Licht umfassen, das von Referenzproben normalen gesunden Gewebes ausgesendet worden ist.

32. Verfahren nach einem der Ansprüche 28 bis 31, wobei die Einträge Einträge von berechneten geschätzten Spektraleigenschaften von Licht umfassen, das von Gewebeproben mit identifizierten Chromophoren zurückgesendet worden ist.

33. Verfahren nach einem der Ansprüche 28 bis 32, ferner umfassend die folgenden Schritte:

Herleiten von Daten, die sich auf das Vorliegen, die Tiefe und die Konzentration von Chromophoren beziehen, und
Erschaffen und Anzeigen einer Karte davon.

34. Verfahren nach einem der Ansprüche 28 bis 33, umfassend
Herleiten von Daten, die sich auf das Vorliegen, die Tiefe und die Konzentration von Chromophoren beziehen, die beliebig aus der folgenden Gruppe ausgewählt worden sind: Melanin, Blut, Haemoglobin, Oxyhaemoglobin, Bilirubin, Tätowierungspigmenten oder -farbstoffen, Keratin, Collagen und Haar.

35. Verfahren nach einem der Ansprüche 28 bis 34, ferner umfassend: Durchlaufenlassen von projeziertem Licht und/oder dem zurückgesendeten Licht durch einen oder mehrere Filter und spektroskopisches Analysieren des zurückgesendeten Lichts, so daß das Analyseergebnis unabhängig vom Vorliegen eines vorbestimmten Chromophors im Gewebe ist.

36. Verfahren nach Anspruch 35, wobei die Lichtquelle (1) und der Photoempfänger (6) dazu ausgelegt sind, Licht mit Wellenlängen zu projizieren und zu detektieren, die im wesentlichen vom Vorliegen des vorbestimmten Chromophors unbeeinflußt sind.

37. Verfahren nach Anspruch 35 oder 36, ferner umfassend:

Verarbeiten von Daten, die die Spektraleigenschaften von vom Photoempfänger (6) empfangenen, zurückgesendeten Licht anzeigen, um Signale zu erzeugen, die das Vorliegen von Chromophoren anzeigen, die im wesentlichen unabhängig von der Variation der Spektraleigenschaften, die aufgrund des Vorliegens der vorbestimmten Chromophors auftreten, sind, und
Vergleichen der verarbeiteten Daten mit den Einträgen, um den Eintrag zu identifizieren, der mit den entsprechenden Spektraldaten am meisten übereinstimmt.

38. Verfahren nach Anspruch 35, 36 oder 37, wobei der vorgegebene Chromophor Melanin, Blut oder Tätowierungspigmente umfaßt.

39. Verfahren nach einem der Ansprüche 28 bis 38, ferner umfassend:

Bestimmen der Tiefe von papillarer Dermis einer überwachten Gewebeprobe auf der Grundlage der Spektraleigenschaften des empfangenen, zurückgesendeten Lichts, und
Identifizieren des Vorliegens einzelner Chromophore innerhalb der Fläche der beleuchteten Gewebeprobe, von der zurückgesendetes Licht empfangen wird, mittels der Spektraleigenschaften des empfangenen, zurückgesendeten Lichts und der bestimmten Tiefe papillarer Dermis.

**Revendications**

1. Appareil pour vérifier la présence de chromophores individuels dans un échantillon de tissu, ledit appareil comprenant :

   - une source de lumière (1) qui est susceptible de fonctionner afin d'illuminer une zone d'un échantillon de tissu avec des longueurs d'onde différentes de lumière ;
   - un photorécepteur (6) disposé de manière à recevoir la lumière réémise par une zone d'un échantillon de tissu illuminé par ladite source de lumière (1) ; et
   - un analyseur spectroscopique (7, 8) qui peut être appliqué pour utiliser les propriétés spectrales de la lumière réémise reçue par ledit photorécepteur (6) et les propriétés spectrales de lumière projetée par ladite source de lumière (1) pour identifier la présence dudit un chromophores ou desdits plusieurs chromophores dans la zone de l'échantillon de tissu illuminé duquel la lumière réémise est reçue par ledit photorécepteur (6),

   **caractérisé en ce que** ledit analyseur spectroscopique (7, 8) comprend :

   - des enregistrements des propriétés spectrales de la lumière réémise par des échantillons de référence de tissu comprenant des chromophores identifiés ;

   ledit analyseur spectroscopique (7, 8) étant disposé de manière à comparer les propriétés spectrales de la lumière réémise reçue par ledit photorécepteur (6) avec lesdits enregistrements pour identifier l'enregistrement comprenant les propriétés spectrales correspondant au mieux aux propriétés de ladite lumière réémise reçue par ledit photorécepteur (6) et pour émettre sous la forme d'une identification de chromophores individuels présents dans la zone de l'échantillon de tissu illuminé duquel la lumière réémise est reçue par ledit photorécepteur (6) une identification des chromophores associés aux dites propriétés spectrales par ledit enregistrement.

2. Appareil selon la revendication 1, dans lequel lesdits enregistrements comprennent les enregistrements des propriétés spectrales de la lumière réémise par les échantillons de référence de tissu épithélial ou de tissu épithélial et sous-épithélial.

3. Appareil selon la revendication 2, dans lequel lesdits enregistrements comprennent les enregistrements des propriétés spectrales de la lumière réémise par lesdits échantillons de référence de peau.

4. Appareil selon l'une quelconque des revendications précédentes, dans lequel lesdits enregistrements comprennent les enregistrements des propriétés spectrales de la lumière réémise par les échantillons de référence de tissu normal sain.

5. Appareil selon l'une quelconque des revendications précédentes, dans lequel lesdits enregistrements comprennent les enregistrements des propriétés spectrales calculées et estimées de la lumière réémise par des échantillons de tissu contenant des chromophores identifiés.

6. Appareil selon l'une quelconque des revendications précédentes dans lequel ladite source de lumière (1) est susceptible de fonctionner afin d'illuminer de manière sélective une zone d'un échantillon de tissu avec différentes longueurs d'onde de lumière.

7. Appareil selon la revendication 6, dans lequel ladite source de lumière (1) comprend en outre un ensemble de filtres (31 à 34) pour une substitution sélective sur le parcours de la lumière incidente au tissu afin d'illuminer de manière sélective une zone de tissu avec différentes longueurs d'onde de lumière.

8. Appareil selon l'une quelconque des revendications précédentes, dans lequel ladite source de lumière (1) comprend en outre des moyens (41) pour polariser la lumière qui illumine ladite zone de tissu.

9. Appareil selon la revendication 8, comprenant en outre des moyens (51) pour polariser de manière croisée la lumière réémise de ladite zone de tissu avant qu'elle soit reçue par ledit photorécepteur (6).

**10.** Appareil selon l'une quelconque des revendications précédentes, dans lequel ladite source de lumière (1) est susceptible de fonctionner afin d'illuminer ladite zone de tissu avec une lumière ayant une longueur d'onde supérieure à 600nm.

**11.** Appareil selon l'une quelconque des revendications précédentes, dans lequel ledit photorécepteur (6) peut être utilisé pour détecter la lumière réémise ayant des longueurs d'onde situées dans la bande de 800 à 1 000 nm et la bande de 600 à 800 nm.

**12.** Appareil selon l'une quelconque des revendications précédentes, dans lequel ledit photorécepteur (6) peut être utilisé pour recevoir la lumière réémise d'une matrice de points et ledit analyseur spectroscopique (7, 8) peut être utilisé pour identifier la présence d'un ou de plusieurs chromophores individuels en chacun desdits points dans ladite matrice.

**13.** Appareil selon la revendication 12, dans lequel ledit photorécepteur (6) peut être utilisé pour recevoir la lumière réémise des points avec une résolution d'au moins 20 lignes ou points par mm.

**14.** Appareil selon l'une quelconque des revendications précédentes, dans lequel ledit analyseur spectroscopique (7, 8) peut être utilisé pour déterminer sur la base des propriétés spectrales de ladite lumière réémise reçue la profondeur du corps papillaire d'un échantillon de tissu étant étudié et pour identifier la présence dudit un chromophores ou desdits plusieurs chromophores individuels dans la zone de l'échantillon de tissu illuminé duquel la lumière réémise est reçue par ledit photorécepteur (6) utilisant les propriétés spectrales de ladite lumière réémise reçue et ladite profondeur déterminée du corps papillaire.

**15.** Appareil selon l'une quelconque des revendications précédentes, dans lequel ladite source de lumière (1), ledit photorécepteur (6) et ledit analyseur spectroscopique (7, 8) sont conjointement adaptés pour générer un signal indicatif de la présence de chromophores qui est essentiellement indépendante de la modification des propriétés spectrales de lumière réémise liée à la présence d'un chromophore prédéterminé.

**16.** Appareil selon la revendication 15 dans lequel ladite source de lumière (1) et ledit photorécepteur (6) sont disposés de manière à projeter et détecter la lumière à des longueurs d'onde qui sont essentiellement insensibles à la présence d'un chromophore prédéterminé.

**17.** Appareil selon la revendication 15 ou la revendication 16, dans lequel ledit analyseur spectroscopique (7, 8) est disposé de manière à traiter les données indicatives des propriétés spectrales de la lumière réémise reçue par ledit photorécepteur (6) pour générer des signaux qui sont indicatifs de la présence de chromophores qui sont essentiellement indépendants de la modification des propriétés spectrales liée à la présence d'un chromophore prédéterminé.

**18.** Appareil selon la revendication 15, la revendication 16 ou la revendication 17, dans lequel ledit chromophore prédéterminé comprend l'un parmi la mélanine, le sang ou des pigments de tatouage.

**19.** Appareil selon l'une quelconque des revendications précédentes, comprenant en outre un guide de lumière dont une partie au moins est flexible utilisée pour conduire la lumière entre ladite source de lumière (1), ledit échantillon de tissu et ledit photorécepteur (6).

**20.** Appareil selon la revendication 16, dans lequel ledit guide de lumière comprend un endoscope.

**21.** Appareil selon la revendication 17, dans lequel ledit guide de lumière se termine en une tête adaptée de manière à être placée contre une zone de la peau.

**22.** Appareil selon l'une quelconque des revendications précédentes, dans lequel les moyens sont fournis pour faire varier la taille de la zone de tissu duquel la lumière réémise est reçue.

**23.** Appareil selon l'une quelconque des revendications précédentes, dans lequel ladite source de lumière (1) est susceptible de fonctionner afin de projeter la lumière qui présente une longueur d'onde suffisamment éloignée dans l'infrarouge pour que son absorption par la mélanine et le sang est négligeable, ou qui présente au moins deux longueurs d'onde dont au moins une est supérieure à 600 nm, dans lequel ledit analyseur spectroscopique (7, 8) peut être utilisé de manière à traiter les données indicatives des propriétés spectrales de la lumière réémise reçue par ledit photorécepteur pour déterminer les modifications des propriétés spectrales provoquées indépendamment par les modifications liées à la présence de mélanine et de sang et de manière à utiliser lesdites modifications indépendantes déterminées des propriétés spectrales pour générer un signal indicatif de la concentration de collagène dans le corps papillaire d'un échantillon de tissu étant contrôlé.

24. Appareil selon l'une quelconque des revendications précédentes comprenant en outre des moyens pour générer des signaux de contrôle sur la base de la détermination de la présence d'un ou de plusieurs chromophores dans un échantillon de tissu par ledit analyseur spectroscopique (7, 8), dans lequel lesdits signaux de contrôle peut être utilisé pour contrôler l'un parmi un moniteur d'affichage (9), une imprimante (10) ou un laser médical (12) ou un autre dispositif ou appareil de traitement.

25. Utilisation de l'appareil selon l'une quelconque des revendications précédentes pour dériver des données concernant la présence, la profondeur et la concentration de chromophores et la création et l'affichage d'une carte de celles-ci.

26. Utilisation de l'appareil selon l'une quelconque des revendications 1 à 24 pour dériver des données concernant la présence, la profondeur et la concentration de l'un quelconque des chromophores choisis dans le groupe comprenant : la mélanine, le sang, l'hémoglobine, l'oxyhémoglobine, la bilirubine, les pigments ou les colorants de tatouage, la kératine, le collagène et les cheveux.

27. Utilisation de l'appareil selon l'une quelconque des revendications 1 à 24 pour tracer une carte de l'étendue d'un épithélioma baso-cellulaire.

28. Procédé de contrôle de la présence de chromophores individuels dans un échantillon de tissu, comprenant :

   - le stockage des enregistrements des propriétés spectrales de lumière réémise par des échantillons de référence de tissu contenant des chromophores identifiés ;
   - l'illumination d'une zone d'un échantillon de tissu avec différentes longueurs d'onde de lumière au moyen d'une source de lumière (1) ;
   - la réception de la lumière réémise par la zone illuminée de tissu à un photorécepteur (6) ;
   - la comparaison des propriétés spectrales de la lumière réémise reçue par ledit photorécepteur (6) avec lesdits enregistrements pour identifier l'enregistrement comprenant les propriétés spectrales correspondant au mieux aux propriétés de ladite lumière réémise reçue par ledit photorécepteur (6) ; et
   - l'émission sous la forme d'une identification des chromophores individuels présents dans la zone de l'échantillon de tissu illuminé duquel la lumière réémise est reçue par ledit photorécepteur (6) une identification des chromophores associés aux dites propriétés spectrales par ledit enregistrement.

29. Procédé selon la revendication 28, dans lequel lesdits enregistrements comprennent les enregistrements des propriétés spectrales de lumière réémise par les échantillons de référence de tissu épithélial ou de tissu épithélial et sous-épithélial.

30. Procédé selon la revendication 29, dans lequel lesdits enregistrements comprennent les enregistrements des propriétés spectrales de lumière réémise par les échantillons de référence de peau.

31. Procédé selon la revendication 28, la revendication 29 ou la revendication 30, dans lequel lesdits enregistrements comprennent les enregistrements des propriétés spectrales de lumière réémise par les échantillons de référence de tissu normal sain.

32. Procédé selon l'une quelconque des revendications 28 à 31, dans lequel lesdits enregistrements comprennent les enregistrements des propriétés spectrales calculées et estimées de la lumière réémise par les échantillons de tissu contenant les chromophores identifiés.

33. Procédé selon l'une quelconque des revendications 28 à 32, comprenant en outre les étapes suivantes:

   - la dérivation des données concernant la présence, la profondeur et la concentration des chromophores ; et
   - la création et l'affichage d'une carte de celles-ci.

34. Procédé selon l'une quelconque des revendications 28 à 33, comprenant : la dérivation des données concernant la présence, la profondeur et la concentration de l'un quelconque chromophore choisi dans le groupe comprenant :

la mélanine, le sang, l'hémoglobine, l'oxyhémoglobine, la bilirubine, des pigments ou des colorants de tatouage, la kératine, le collagène et les cheveux.

35. Procédé selon l'une quelconque des revendications 28 à 34 qui comprend en outre : le passage de la lumière projetée et/ou la lumière réémise à travers un ou plusieurs filtres et l'analyse spectroscopique de la lumière réémise de sorte que le résultat de l'analyse est indépendant de la présence d'un chromophore prédéterminé dans le tissu.

36. Procédé selon la revendication 35 dans lequel ladite source de lumière

(1) et le photorécepteur (6) sont disposés de manière à projeter et détecter la lumière à des longueurs d'onde qui sont essentiellement insensibles à la présence dudit chromophore prédéterminé.

37. Procédé selon la revendication 35 ou la revendication 36 comprenant en outre :

- le traitement des données indicatives des propriétés spectrales de la lumière réémise reçue par ledit photorécepteur (6) pour générer des signaux qui sont indicatifs de la présence de chromophores qui sont essentiellement indépendants de la modification des propriétés spectrales liée à la présence desdits chromophores prédéterminés ; et
- la comparaison des dites données traitées avec lesdits enregistrements pour identifier l'enregistrement ayant les données spectrales correspondant au mieux.

38. Procédé selon la revendication 35, la revendication 36 ou la revendication 37, dans lequel ledit chromophore prédéterminé comprend l'un quelconque parmi la mélanine, le sang et les pigments de tatouage.

39. Procédé selon l'une quelconque des revendications 28 à 38, comprenant en outre :

- la détermination de la profondeur du corps papillaire d'un échantillon de tissu étant contrôlé sur la base des propriétés spectrales de ladite lumière réémise reçue ; et
- l'identification de la présence de chromophores individuels dans la zone de l'échantillon de tissu illuminé duquel la lumière réémise est reçue en utilisant les propriétés spectrales de ladite lumière réémise reçue et ladite profondeur déterminée du corps papillaire.

**Fig. 1**

Epidermis

Rete pegs

max1

Dermal papillae

min1

Papillary dermis

$V_1$ $V_2$ $V_3$

C1

*Fig. 2*

Reticular dermis

*Fig. 10*

Epidermis                    Rete ridges

Papillary dermis            Dermal papillae

Reticular dermis

## Fig. 3

Epidermis

Blister

Papillary dermis            Dermal papillae

## Fig. 4

Epidermis      Blister

Papillary dermis            Dermal papillae

## Fig. 5

## Fig. 6

## Fig. 7

*Fig. 8*

Fig. 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9822023 A

- US 5701902 A

**Non-patent literature cited in the description**

- **R. ANDERSON, ; B. PARRISH ; J. PARRISH.** The optics of human skin. *The Journal of Investigative Dermatology*
- A non-invasive imaging system for assisting in the diagnosis of melanoma. *Symon Cotton,* 1998

- The papillary layer consists of loose connective tissue. **MICHAEL ROSS ; LYNN ROMRELL.** Histology, a text and atlas